(19) Europäisches Patentamt
European Patent Office
Office européen des brevets

(11) Veröffentlichungsnummer: **0 242 829 A1**

(12) **EUROPÄISCHE PATENTANMELDUNG**

(21) Anmeldenummer: **87105748.5**

(22) Anmeldetag: **17.04.87**

(51) Int. Cl.$^4$: **C07D 211/90**, C07D 405/14, C07D 409/14, C07D 413/14, C07D 417/14, A61K 31/44, //C07D211/14

Die Bezeichnung der Erfindung wurde geändert (Richtlinien für die Prüfung im EPA, A-III, 7.3).

(30) Priorität: **22.04.86 CH 1633/86**
**22.04.86 CH 1623/86**

(43) Veröffentlichungstag der Anmeldung:
**28.10.87 Patentblatt 87/44**

(84) Benannte Vertragsstaaten:
**ES GR**

(71) Anmelder: **Byk Gulden Lomberg Chemische Fabrik GmbH**
**Byk-Gulden-Strasse 2**
**D-7750 Konstanz(DE)**

(72) Erfinder: **Amschler, Hermann**
**Hohenhewenstrasse 19**
**D-7760 Radolfzell(DE)**
Erfinder: **Eistetter, Klaus, Dr.**
**Säntisblick 7**
**D-7750 Konstanz 19(DE)**
Erfinder: **Eltze, Manfrid, Dr.**
**Schützenstrasse 20**
**D-7750 Konstanz(DE)**
Erfinder: **Flockerzi, Dieter, Dr.**
**Ackerweg 26**
**D-7753 Allensbach(DE)**
Erfinder: **Klemm, Kurt, Prof. Dr.**
**Im Weinberg 2**
**D-7753 Allensbach(DE)**
Erfinder: **Kolassa, Norbert, Prof. Dr.**
**Lerchenweg 12**
**D-7750 Konstanz(DE)**
Erfinder: **Sanders, Karl, Dr.**
**Felchengang 23**
**D-7750 Konstanz(DE)**
Erfinder: **Schudt, Christian, Dr.**
**Hoheneggstrasse 112**
**D-7750 Konstanz(DE)**
Erfinder: **Ulrich, Wolf-Rüdiger, Dr.**
**Hebelstrasse 3**
**D-7750 Konstanz(DE)**

(54) **Dihydropyridine, Verfahren zu ihrer Herstellung und ihre Verwendung als Arzneimittel.**

(57) Dihydropyridine der Formel I,

EP 0 242 829 A1

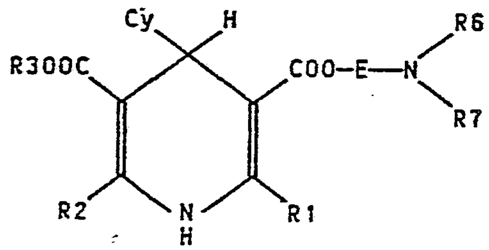

(I)

worin
R6 und R7 gemeinsam und unter Einschluß des Stickstoffatoms, an das beide gebunden sind, einen Rest der Formel

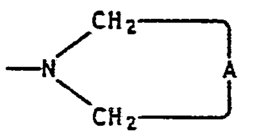

darstellen, worin
A $-CH_2-C(R8)R9-CH_2-$bedeutet,
R8 Aryl bedeutet und
R9 Aryl bedeutet, wobei
Aryl für einen Ring der Formel

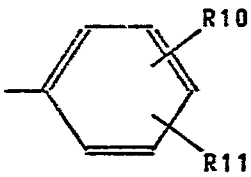

steht, in dem R10 und R11 gleich oder verschieden sind und die Bedeutung Wasserstoff (H), 1-4C-Alkyl, 1-4C-Alkoxy, Halogen, Hydroxy oder Trifluormethyl haben,
und worin
entweder
E 2-5C-Alkylen,
R2 Amino ($NH_2$) und
R3 1-6C-Alkyl oder 3-7C-Alkoxyalkyl bedeuten,
oder
E A1-O-A2,
R2 Wasserstoff, 1-6C-Alkyl oder 3-7C-Alkoxyalkyl und
R3 Wasserstoff, 1-6C-Alkyl oder 3-7C-Alkoxyalkyl bedeuten,
wobei
A1 2-4C-Alkylen und
A2 2-4C-Alkylen oder 2C-Alkylenoxy-2C-alkylen bedeutet,
und die Salze dieser Verbindungen, Verfahren zu ihrer Herstellung und ihre Verwendung als Arzneimittel.

# Neue Amine und Ether

## Anwendungsgebiet der Erfindung

Die Erfindung betrifft neue Amine und Ether, Verfahren zu ihrer Herstellung, ihre Verwendung und sie enthaltende Arzneimittel. Die erfindungsgemäßen Verbindungen werden in der pharmazeutischen Industrie zur Herstellung von Arzneimitteln eingesetzt.

## Bekannter technischer Hintergrund

Es ist bekannt, daß bestimmte, auf verschiedene Weise substituierte 1,4-Dihydropyridinderivate pharmakologisch nützliche Eigenschaften aufweisen. Überraschenderweise wurde nun gefunden, daß die unten näher beschriebenen neuen Verbindungen besonders interessante pharmakologische Eigenschaften aufweisen, durch die sie sich von den Verbindungen des Standes der Technik in vorteilhafter Weise unterscheiden.

## Beschreibung der Erfindung

Gegenstand der Erfindung sind neue Amine und Ether der Formel I

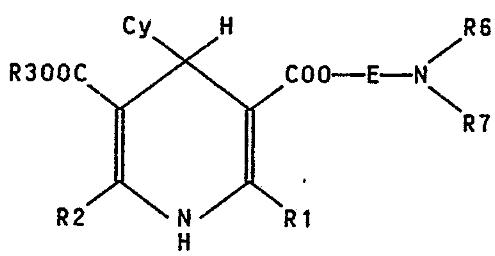

(I)

worin Cy einen Cyclus der Formel

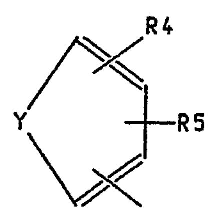

darstellt, in dem Y Sauerstoff (O), Schwefel (S), Vinylen (-CH = CH-), Azomethin (-CH = N-) oder eine Gruppe der Formel

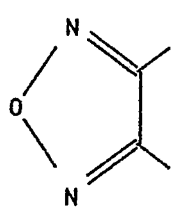

oder

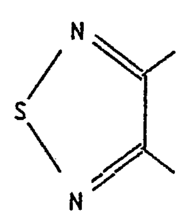

bedeutet,

R1 Wasserstoff, 1-6C-Alkyl oder 3-7C-Alkoxyalkyl bedeutet,

R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxycarbonyl, 2-5C-Acyl, Amino oder Mono-oder Di-1-4C-alkylamino bedeuten,

R6 und R7 gemeinsam und unter Einschluß des Stickstoffatoms, an das beide gebunden sind, einen Rest der Formel

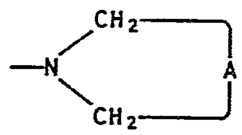

darstellen, worin

A -$CH_2$-C(R8)R9-$CH_2$-bedeutet,

R8 Aryl bedeutet und

R9 Aryl bedeutet, wobei

Aryl für einen Ring der Formel

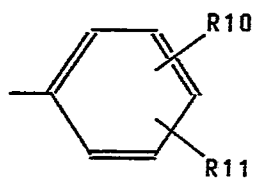

steht, in dem R10 und R11 gleich oder verschieden sind und die Bedeutung Wasserstoff (H), 1-4C-Alkyl, 1-4C-Alkoxy, Halogen, Hydroxy oder Trifluormethyl haben,

und worin

entweder

E 2-5C-Alkylen,

R2 Amino ($NH_2$) und

R3 1-6C-Alkyl oder 3-7C-Alkoxyalkyl bedeuten,

oder

E A1-O-A2,

R2 Wasserstoff, 1-6C-Alkyl oder 3-7C-Alkoxyalkyl und

R3 Wasserstoff, 1-6C-Alkyl oder 3-7C-Alkoxyalkyl bedeuten,

wobei

A1 2-4C-Alkylen und

A2 2-4C-Alkylen oder 2C-Alkylenoxy-2C-alkylen bedeutet,

und die Salze dieser Verbindungen.

1-6C-Alkyl ist geradkettig oder verzweigt und bedeutet beispielsweise einen Hexyl-, Neopentyl-, Isopentyl-, Butyl-, i-Butyl-, sec.-Butyl-, t-Butyl-, Propyl-, Isopropyl-oder insbesondere Ethyl-oder Methylrest.

3-7C-Alkoxyalkyl steht beispielsweise für einen Ethoxyethyl-, Propoxyethyl-, Isopropoxyethyl-, Butoxyethyl-, Methoxypropyl-, 2-Methoxy-1-methylethyl-, 2-Ethoxy-1-methylethyl-oder insbesondere Methoxyethylrest.

Halogen im Sinne der Erfindung bedeutet Brom, Fluor und insbesondere Chlor.

1-4C-Alkyl ist geradkettig oder verzweigt und bedeutet beispielsweise einen Butyl-, i-Butyl-, sec.-Butyl-, t-Butyl-, Propyl-, Isopropyl-, Ethyl-oder insbesondere Methylrest.

1-4C-Alkoxy enthält neben dem Sauerstoffatom einen der vorstehend genannten 1-4C-Alkylreste. Bevorzugt sind der Methoxy-und der Ethoxyrest.

Ganz oder teilweise durch Fluor substituiertes 1-4C-Alkoxy ist beispielsweise 1,1,2,2-Tetrafluorethoxy, Trifluormethoxy, 2,2,2,-Trifluorethoxy oder insbesondere Difluormethoxy.

1-4C-Alkoxycarbonyl enthält neben der Carbonylgruppe einen der vorstehend genannten 1-4C-Alkoxyreste. Bevorzugt sind der Methoxycarbonyl-und der Ethoxycarbonylrest.

2-5C-Acyl enthält neben der Carbonylgruppe einen der vorstehend genannten 1-4C-Alkylreste. Bevorzugt ist der Acetylrest.

Mono-oder Di-1-4C-alkylamino enthält neben dem Stickstoffatom einen oder zwei der vorstehend genannten 1-4C-Alkylreste. Bevorzugt ist Di-1-4C-alkylamino, und hier insbesondere Dimethyl-, Diethyl-oder Diisopropylamino.

Aryl steht für durch R10 und R11 substituiertes Phenyl. Als beispielhafte bevorzugte Arylreste seien genannt die Reste: Phenyl, 4-Methoxyphenyl, 4-Chlorphenyl, 4-Methylphenyl, 4-Fluorphenyl, 3-Fluorphenyl, 3-Chlorphenyl, 2-Chlorphenyl, 3-Methoxyphenyl, 2-Methoxyphenyl, 2-Methylphenyl, 3-Chlor-4-methylphenyl, 3,4-Dichlorphenyl, 3,6-Dichlorphenyl, 3,4-Dimethylphenyl, 2-Trifluormethylphenyl und 3-Trifluormethylphenyl.

2-5C-Alkylen ist geradkettig oder verzweigt und steht beispielsweise für Ethylen (-$CH_2$-$CH_2$-), Trimethylen (-$CH_2$-$CH_2$-$CH_2$-), Tetramethylen (-$CH_2$-$CH_2$-$CH_2$-$CH_2$-), Pentamethylen (-$CH_2$-$CH_2$-$CH_2$-$CH_2$-$CH_2$-), 1,2-Dimethylethylen, 1,1-Dimethylethylen, 2,2-Dimethylethylen, Isopropyliden, 1-Methylethylen und 2-Ethylpropylen, wobei Ethylen und Trimethylen bevorzugt sind.

2-4C-Alkylen steht für Ethylen (-$CH_2$-$CH_2$-), Trimethylen (-$CH_2$-$CH_2$-$CH_2$-) und Tetramethylen (-$CH_2$-$CH_2$-$CH_2$-$CH_2$-), wobei Ethylen bevorzugt ist.

2-C-Alkylenoxy-2C-alkylen steht für Ethylen, das durch Ethylenoxy substituiert ist (-$CH_2$-$CH_2$-O-$CH_2$-$CH_2$-).

Als Salze kommen alle Salze mit Säuren in Betracht. Besonders erwähnt seien die pharmakologisch verträglichen Salze der in der pharmazeutischen Industrie üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Metembonat, Stearat, Tosilat, 2-Hydroxy-3-naphthoat, 3-Hydroxy-2-naphthoat oder Mesilat, aber auch Salze mit Bumetanid, Furosemid, Azosemid, Galosemid, Besunid, Piretanid, Etacrynsäure, Tienilinsäure oder 4-Chlor-sulfamoyl-benzoesäure.

Eine Ausgestaltung (Ausgestaltung a) der Erfindung sind Verbindungen der Formel Ia

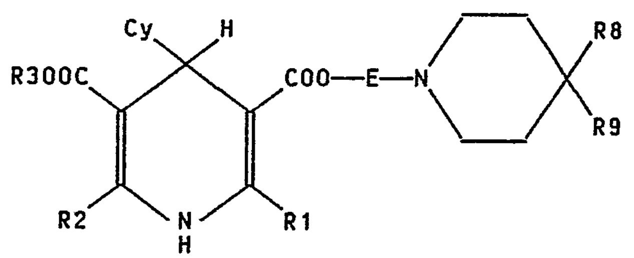

(Ia)

worin Cy einen Cyclus der Formel

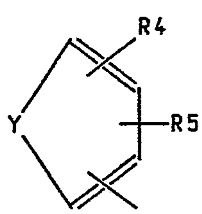

darstellt, in dem Y Sauerstoff (O), Schwefel (S), Vinylen (-CH=CH-), Azomethin (-CH=N-) oder eine Gruppe der Formel

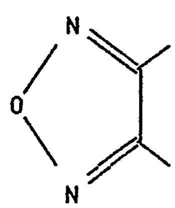

oder

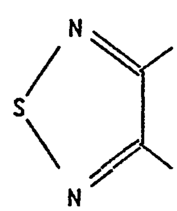

bedeutet,

E 2-5C-Alkylen bedeutet,

R1 Wasserstoff, 1-6C-Alkyl oder 3-7C-Alkoxyalkyl bedeutet,

R2 Amino ($NH_2$) bedeutet,

R3 1-6C-Alkyl oder 3-7C-Alkoxyalkyl bedeutet,

R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxycarbonyl, 2-5C-Acyl, Amino oder Mono-oder Di-1-4C-alkylamino bedeuten,

R8 Aryl bedeutet und

R9 Aryl bedeutet, wobei

Aryl für einen Ring der Formel

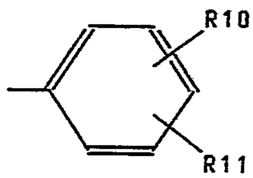

steht, worin R10 und R11 gleich oder verschieden sind und die Bedeutung Wasserstoff (H), 1-4C-Alkyl, 1-4C-Alkoxy, Halogen, Hydroxy oder Trifluormethyl haben,

und die Salze dieser Verbindungen.

Hervorzuhebende erfindungsgemäße Verbindungen der Ausgestaltung a sind solche der Formel Ia, worin

Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl oder Benzoxdiazolyl bedeutet,

E Ethylen ($-CH_2CH_2-$), Trimethylen ($-CH_2-CH_2-CH_2-$) oder Pentamethylen ($-CH_2-CH_2-CH_2-CH_2-CH_2-$) bedeutet,

R1 Methyl bedeutet,

R2 Amino ($NH_2$) bedeutet,

R3 Methyl, Ethyl oder Methoxyethyl bedeutet,

R8 Aryl bedeutet und

R9 Aryl bedeutet, wobei

Aryl für einen Ring der Formel

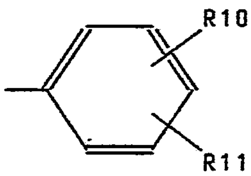

steht, worin R10 und R11 gleich oder verschieden sind und die Bedeutung Wasserstoff (H), Methyl, Methoxy, Chlor, Fluor, Hydroxy oder Trifluormethyl haben, und die Salze der Verbindungen.

Bevorzugte erfindungsgemäße Verbindungen der Ausgestaltung a sind solche der Formel Ia, worin

Cy 3-Nitrophenyl, 2-Chlorphenyl, 2,3-Dichlorphenyl, 2-Trifluormethylphenyl oder Benzoxdiazolyl bedeutet,

E Ethylen ($-CH_2-CH_2-$), Trimethylen ($-CH_2-CH_2-CH_2-$) oder Pentamethylen ($-CH_2-CH_2-CH_2-CH_2-CH_2-$) bedeutet,

R1 Methyl bedeutet,

R2 Amino ($NH_2$) bedeutet,

R3 Methyl oder Ethyl bedeutet,

R8 Phenyl bedeutet und

R9 Phenyl bedeutet,

und ihre Salze.

Besonders bevorzugte erfindungsgemäße Verbindungen der Ausgestaltung a sind solche der Formel Ia, worin

Cy 3-Nitrophenyl bedeutet,

E Ethylen ($-CH_2-CH_2-$) oder Trimethylen ($-CH_2-CH_2-CH_2-$) bedeutet,

R1 Methyl bedeutet,

R2 Amino ($NH_2$) bedeutet,

R3 Methyl oder Ethyl bedeutet,

R8 Phenyl und

R9 Phenyl bedeutet,

und ihre Salze.

Als erfindungsgemäße Verbindungen der Ausgestaltung a seien beispielsweise genannt:

2-Amino-1,4-dihydro-6-methyl-4-(3-nitrophenyl)-pyridin-3,5-dicarbonsäure-3-ethyl-5-[2-(4,4-diphenylpiperidyl-1)-ethyl]-ester,

2-Amino-1,4-dihydro-6-methyl-4-(3-nitrophenyl)-pyridin-3,5-dicarbonsäure-3-(2-methoxyethyl)-5-[3-(4,4-diphenylpiperidyl-1)-propyl]-ester,

2-Amino-1,4-dihydro-6-methyl-4-(3-nitrophenyl)-pyridin-3,5-dicarbonsäure-3-methyl-5-[4-(4,4-diphenylpiperidyl-1]-butyl]-ester,

2-Amino-1,4-dihydro-6-methyl-4-(2-nitrophenyl)-pyridin-3,5-dicarbonsäure-3-methyl-5-[2-(4,4-diphenylpiperidyl-1)-ethyl]-ester,

2-Amino-1,4-dihydro-6-ethyl-4-(3-nitrophenyl)-pyridin-3,5-dicarbonsäure-3-methyl-5-[2-(4,4-diphenylpiperidyl-1)-ethyl]-ester,

2-Amino-1,4-dihydro-6-methyl-4-(3-nitrophenyl)-pyridin-3,5-dicarbonsäure-3-(propyl-2)-5-[2-(4,4-diphenylpiperidyl-1)-ethyl]-ester,

2-Amino-1,4-dihydro-6-methyl-4-(3-nitrophenyl)-pyridin-3,5-dicarbonsäure-3-hexyl-5-[2-(4,4-diphenylpiperidyl-1)-ethyl]-ester,

2-Amino-1,4-dihydro-6-methyl-4-(3-nitrophenyl)-pyridin-3,5-dicarbonsäure-3-(2-n-butoxyethyl)-5-[2-(4,4-diphenylpiperidyl-1)-ethyl]-ester,

2-Amino-1,4-dihydro-6-methyl-4-(3-nitrophenyl)-pyridin-3,5-dicarbonsäure-3-methyl-5-{2-[4,4-di(4-methoxyphenyl)-piperidyl-1]-ethyl}-ester,

2-Amino-1,4-dihydro-6-methyl-4-(2-trifluormethylphenyl)-pyridin-3,5-dicarbonsäure-3-ethyl-5-[2-(4,4-diphenylpiperidyl-1)-ethyl]-ester,

2-Amino-1,4-dihydro-6-methyl-4-[3-(1,1,2,2-tetrafluorethoxy)-phenyl)-pyridin-3,5-dicarbonsäure-3-methyl-5-[2-(4,4-diphenylpiperidyl-1)-ethyl]-ester,

2-Amino-1,4-dihydro-6-methyl-4-(2-difluormethoxyphenyl)-pyridin-3,5-dicarbonsäure-3-ethyl-5-[2-(4,4-diphenylpiperidyl-1)-ethyl]-ester,

2-Amino-1,4-dihydro-6-methyl-4-(2-difluormethoxyphenyl)-pyridin-3,5-dicarbonsäure-3-ethyl-5-[4-(4,4-diphenylpiperidyl-1)-butyl]-ester,

2-Amino-1,4-dihydro-6-methyl-4-(3-nitrophenyl)-pyridin-3,5-dicarbonsäure-3-methyl-5-[2-(4,4-dihydroxyphenylpiperidyl-1)-ethyl]-ester,

2-Amino-4-(2,3-dichlorphenyl)-1,4-dihydro-6-methylpyridin-3,5-dicarbonsäure-3-methyl-5-[2-(4,4-diphenylpiperidyl-1)-ethyl]-ester,

2-Amino-4-(2,1,3-benzoxdiazol-4-yl)-1,4-dihydro-6-methylpyridin-3,5-dicarbonsäure-3-methyl-5-[2-(4,4-diphenylpiperidyl-1)-ethyl]-ester,

2-Amino-4-(3-cyanphenyl)-1,4-dihydro-6-methylpyridin-3,5-dicarbonsäure-3-methyl-5-[2-(4,4-diphenylpiperidyl-1)-ethyl]-ester,

2-Amino-1,4-dihydro-6-methyl-4-(2-methoxyphenyl)-pyridin-3,5-dicarbon säure-3-methyl-5-[2-(4,4-diphenylpiperidyl-1)-ethyl]-ester,

2-Amino-1,4-dihydro-6-methyl-4-(2-pyridyl)-pyridin-3,5-dicarbonsäure3-methyl-5-[2-(4,4-diphenylpiperidyl-1)-ethyl]-ester,

2-Amino-1,4-dihydro-6-methyl-4-(5-methyl-2-thienyl)-pyridin-3,5-dicarbonsäure-3-methyl-5-[2-(4,4-diphenylpiperidyl-1)-ethyl]-ester,

2-Amino-1,4-dihydro-6-methyl-4-(3-nitrophenyl)-pyridin-3,5-dicarbonsäure-3-methyl-5-{2-[4-(4-chlorphenyl)-4-phenylpiperidyl-1]-ethyl}-ester,

2-Amino-1,4-dihydro-6-methyl-4-(2-nitrophenyl)-pyridin-3,5-dicarbonsäure-3-methyl-5-[3-(4,4-diphenylpiperidyl-1)-propyl]-ester,

2-Amino-1,4-dihydro-6-ethyl-4-(3-nitrophenyl)-pyridin-3,5-dicarbonsäure-3-methyl-5-[3-(4,4-diphenylpiperidyl-1)-propyl]-ester,

2-Amino-1,4-dihydro-6-methyl-4-(3-nitrophenyl)-pyridin-3,5-dicarbonsäure-3-(propyl-2)-5-[3-(4,4-diphenylpiperidyl-1)-propyl]-ester,
2-Amino-1,4-dihydro-6-methyl-4-(3-nitrophenyl)-pyridin-3,5-dicarbonsäure-3-hexyl-5-[3-(4,4-diphenylpiperidyl-1)-propyl]-ester,
2-Amino-1,4-dihydro-6-methyl-4-(3-nitrophenyl)-pyridin-3,5-dicarbonsäure-3-(2-n-butoxyethyl)-5-[3-(4,4-diphenylpiperidyl-1)-propyl]-ester,
2-Amino-1,4-dihydro-6-methyl-4-(3-nitrophenyl)-pyridin-3,5-dicarbonsäure-3-methyl-5-{3-[4,4-di(4-methoxyphenyl)-piperidyl-1]-propyl}-ester,
2-Amino-1,4-dihydro-6-methyl-4-(2-trifluormethylphenyl)-pyridin-3,5-dicarbonsäure-3-ethyl-5-[3-(4,4-diphenylpiperidyl-1)-propyl]-ester,
2-Amino-1,4-dihydro-6-methyl-4-[3-(1,1,2,2-tetrafluorethoxy)-phenyl]-pyridin-3,5-dicarbonsäure-3-methyl-5-[3-(4,4-diphenylpiperidyl-1)-propyl]-ester,
2-Amino-1,4-dihydro-6-methyl-4-(2-difluormethoxyphenyl)-pyridin-3,5-dicarbonsäure-3-ethyl-5-[3-(4,4-diphenylpiperidyl-1)-propyl]-ester,
2-Amino-1,4-dihydro-6-methyl-4-(3-nitrophenyl)-pyridin-3,5-dicarbonsäure-3-methyl-5-[3-(4,4-dihydroxyphenylpiperidyl-1)-propyl]-ester,
2-Amino-4-(2,3-dichlorphenyl)-1,4-dihydro-6-methylpyridin-3,5-dicarbonsäure-3-methyl-5-[3-(4,4-diphenylpiperidyl-1)-propyl]-ester,
2-Amino-4-(2,1,3-benzoxdiazol-4-yl)-1,4-dihydro-6-methylpyridin-3,5-dicarbonsäure-3-methyl-5-[3-(4,4-diphenylpiperidyl-1)-propyl]-ester,
2-Amino-4-(3-cyanphenyl)-1,4-dihydro-6-methylpyridin-3,5-dicarbonsäure-3-methyl-5-[3-(4,4-diphenylpiperidyl-1)-propyl]-ester,
2-Amino-1,4-dihydro-6-methyl-4-(2-methoxyphenyl)-pyridin-3,5-dicarbonsäure-3-methyl-5-[3-(4,4-diphenylpiperidyl-1)-propyl]-ester,
2-Amino-1,4-dihydro-6-methyl-4-(2-pyridyl)-pyridin-3,5-dicarbonsäure3-methyl-5-[3-(4,4-diphenylpiperidyl-1)-propyl]-ester,
2-Amino-1,4-dihydro-6-methyl-4-(5-methyl-2-thienyl)-pyridin-3,5-dicarbonsäure-3-methyl-5-[3-(4,4-diphenylpiperidyl-1)-propyl]-ester und
2-Amino-1,4-dihydro-6-methyl-4-(3-nitrophenyl)-pyridin-3,5-dicarbonsäure-3-methyl-5-{3-[4-(4-chlorphenyl)-4-phenylpiperidyl-1]-propyl}-ester
und ihre Salze.

Eine weitere Ausgestaltung (Ausgestaltung b) der Erfindung sind Verbindungen der Formel Ib

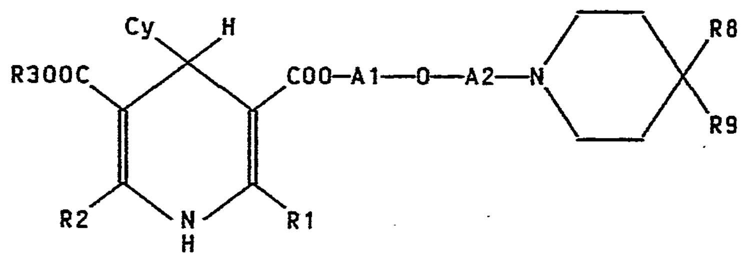

(Ib)

worin Cy einen Cyclus der Formel

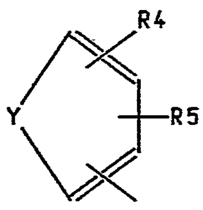

darstellt, in dem Y Sauerstoff (O), Schwefel (S), Vinylen (-CH = CH-), Azomethin (-CH = N-) oder eine Gruppe der Formel

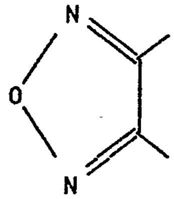

oder

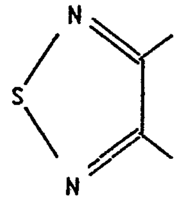

bedeutet,

A1 2-4C-Alkylen bedeutet,

A2 2-4C-Alkylen oder 2C-Alkylenoxy-2C-alkylen bedeutet,

R1 und R2 gleich oder verschieden sind und Wasserstoff, 1-6C-Alkyl oder 3-7C-Alkoxyalkyl bedeuten,

R3 Wasserstoff, 1-6C-Alkyl oder 3-7C-Alkoxyalkyl bedeutet,

R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxycarbonyl, 2-5C-Acyl, Amino oder Mono-oder Di-1-4C-alkylamino bedeuten,

R8 Aryl bedeutet und

R9 Aryl bedeutet, wobei

Aryl für einen Ring der Formel

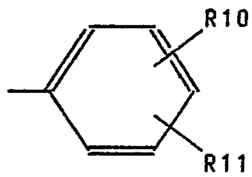

steht, worin R10 und R11 gleich oder verschieden sind und die Bedeutung Wasserstoff (H), 1-4C-Alkyl, 1-4C-Alkoxy, Halogen, Hydroxy oder Trifluormethyl haben,
und die Salze dieser Verbindungen.

Hervorzuhebende erfindungsgemäße Verbindungen der Ausgestaltung b sind solche der Formel Ib, worin

Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl oder Benzoxdiazolyl bedeutet,

A1 Ethylen ($-CH_2CH_2-$) bedeutet,

A2 Ethylen ($-CH_2CH_2-$) oder Ethylenoxyethylen ($-CH_2-CH_2-O-CH_2--CH_2-$) bedeutet,

R1 Methyl bedeutet,

R2 Methyl bedeutet,

R3 Methyl, Ethyl oder Methoxyethyl bedeutet,

R8 Aryl bedeutet und

R9 Aryl bedeutet, wobei

Aryl für einen Ring der Formel

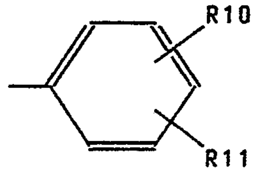

steht, worin R10 und R11 gleich oder verschieden sind und die Bedeutung Wasserstoff (H), Methyl, Methoxy, Chlor, Fluor, Hydroxy oder Trifluormethyl haben, und die Salze der Verbindungen.

Bevorzugte erfindungsgemäße Verbindungen der Ausgestaltung b sind solche der Formel Ib, worin

Cy 3-Nitrophenyl, 2-Chlorphenyl, 2,3-Dichlorphenyl, 2-Trifluormethylphenyl oder Benzoxdiazolyl bedeutet,

A1 Ethylen ($-CH_2CH_2-$) bedeutet,

A2 Ethylen ($-CH_2CH_2-$) oder Ethylenoxyethylen ($-CH_2CH_2-O-CH_2-CH_2-$) bedeutet,

R1 Methyl bedeutet,
R2 Methyl bedeutet,
R3 Methyl oder Ethyl bedeutet
R8 Phenyl bedeutet und
R9 Phenyl bedeutet,
und ihre Salze.

Besonders bevorzugte erfindungsgemäße Verbindungen der Ausgestaltung b sind solche der Formel Ib, worin

Cy 3-Nitrophenyl bedeutet
A1 Ethylen ($-CH_2CH_2-$) bedeutet,
A2 Ethylen ($-CH_2CH_2-$) bedeutet,
R1 Methyl bedeutet,
R2 Methyl bedeutet,
R3 Methyl bedeutet,
R8 Phenyl und
R9 Phenyl bedeutet,
und ihre Salze.

Als erfindungsgemäße Verbindungen der Ausgestaltung b seien beispielsweise genannt:

1,4-Dihydro-2,6-dimethyl-4-(3-nitrophenyl)-pyridin-3,5-dicarbonsäure-3-ethyl-5-{2-[2-(4,4-diphenylpiperidyl-1)-ethoxi]-ethyl}-ester,
1,4-Dihydro-2,6-dimethyl-4-(3-nitrophenyl)-pyridin-3,5-dicarbonsäure-3-(2-methoxyethyl)-5-{3-[3-(4,4-diphenylpiperidyl-1)-propoxi]-propyl}-ester,
1,4-Dihydro-2,6-dimethyl-4-(2-nitrophenyl)-pyridin-3,5-dicarbonsäure-3-methyl-5-{2-[2-(4,4-diphenylpiperidyl-1)-ethoxi]-ethyl}-ester,
1,4-Dihydro-2,6-diethyl-4-(3-nitrophenyl)-pyridin-3,5-dicarbonsäure-3-methyl-5-{2-[2-(4,4-diphenylpiperidyl-1)-ethoxi]-ethyl}-ester,
1,4-Dihydro-2,6-dimethyl-4-(3-nitrophenyl)-pyridin-3,5-dicarbonsäure-3-(propyl-2)-5-{2-[2-(4,4-diphenylpiperidyl-1)-ethoxi]-ethyl}-ester,
1,4-Dihydro-2,6-dimethyl-4-(3-nitrophenyl)-pyridin-3,5-dicarbonsäaure-3-hexyl-5-{2-[2-(4,4-diphenylpiperidyl-1)-ethoxi]-ethyl}-ester,
1,4-Dihydro-2,6-dimethyl-4-(3-nitrophenyl)-pyridin-3,5-dicarbonsäure-3-(2-n-butoxyethyl)-5-{2-[2-(4,4-diphenylpiperidyl-1)-ethoxi]-ethyl}-ester,
1,4-Dihydro-2,6-dimethyl-4-(2-trifluoremethylphenyl)-pyridin-3,5-dicarbonsäure-3-ethyl-5-{2-[2-(4,4-diphenylpiperidyl-1)-ethoxi]-ethyl}-ester,
1,4-Dihydro-2,6-dimethyl-4-[3-(1,1,2,2-tetrafluorethoxy)-phenyl]-pyridin-3,5-dicarbonsäure-3-methyl-5-{2-[2-(4,4-diphenylpiperidyl-1)-ethoxi]-ethyl}-ester,
1,4-Dihydro-2,6-dimethyl-4-(2-difluormethoxyphenyl)-pyridin-3,5-dicarbonsäure-3-ethyl-5-{2-[2-(4,4-diphenylpiperidyl-1)-ethoxi]-ethyl}-ester,
1,4-Dihydro-2,6-dimethyl-4-(3-nitrophenyl)-pyridin-3,5-dicarbonsäure-3-methyl-5-{2-[2-(4,4-dihydroxyphenylpiperidyl-1)-ethoxi]-ethyl}-ester,
4-(2,3-Dichlorphenyl)-1,4-dihydro-2,6-dimethylpyridin-3,5-dicarbonsäure-3-methyl-5-{2-[2-(4,4-diphenylpiperidyl-1)-ethoxi]-ethyl}-ester,
4-(2,1,3-Benzoxdiazol-4-yl)-1,4-dihydro-2,6-dimethylpyridin-3,5-dicarbonsäure-3-methyl-5-{2-[2-(4,4-diphenylpiperidyl-1)-ethoxi]-ethyl}-ester,
4-(3-Cyanphenyl)-1,4-dihydro-2,6-dimethylpyridin-3,5-dicarbonsäure-3-methyl-5-{2-[2-(4,4-diphenylpiperidyl-1)-ethoxi]-ethyl}-ester,
1,4-Dihydro-2,6-dimethyl-4-(2-methoxyphenyl)-pyridin-3,5-dicarbonsäure-3-methyl-5-{2-[2-(4,4-diphenylpiperidyl-1)-ethoxi]-ethyl}-ester,
1,4-Dihydro-2,6-dimethyl-4-(2-pyridyl)-pyridin-3,5-dicarbonsäure3-methyl-5-{2-[2-(4,4-diphenylpiperidyl-1)-ethoxi]-ethyl}-ester,
1,4-Dihydro-2,6-dimethyl-4-(5-methyl-2-thienyl)-pyridin-3,5-dicarbonsäure-3-methyl-5-{2-[2-(4,4-diphenylpiperidyl-1)-ethoxi]-ethyl}-ester,
1,4-Dihydro-2,6-dimethyl-4-(2-nitrophenyl)-pyridin-3,5-dicarbonsäure3-me-thyl-5-{3-[3-(4,4-diphenylpiperidyl-1)-propoxi]-propyl}-ester,
1,4-Dihydro-2,6-diethyl-4-(3-nitrophenyl)-pyridin-3,5-dicarbonsäure-methyl-5-{3-[3-(4,4-diphenylpiperidyl-1)-propoxi]-propyl}-ester,
1,4-Dihydro-2,6-dimethyl-4-(3-nitrophenyl)-pyridin-3,5-carbonsäure-3-(propyl-2)-5-{3-[3-(4,4-diphenylpiperidyl-1)-propoxi]-propyl}-ester,

1,4-Dihydro-2,6-dimethyl-4-(3-nitrophenyl)-pyridin-3,5-dicarbonsäure-3-hexyl-5-{3-[3-(4,4-diphenylpiperidyl-1)-propoxi]-propyl}-ester,
1,4-Dihydro-2,6-dimethyl-4-(3-nitrophenyl)-pyridin-3,5-dicarbonsäure-3-(2-n-butoxyethyl)-5-{3-[3-(4,4-diphenylpiperidyl-1)-propoxy]-propyl}-ester,
1,4-Dihydro-2,6-dimethyl-4-(2-trifluormethylphenyl)-pyridin-3,5-dicarbonsäure-3-ethyl-5-{3-[3-(4,4-diphenylpiperidyl-1)-propoxi]-propyl}-ester,
1,4-Dihydro-2,6-dimethyl-4-[3-(1,1,2,2-tetrafluorethoxy)-phenyl]-pyridin-3,5-dicarbonsäure-3-methyl-5-{3-[3-(4,4-diphenylpiperidyl-1)-propoxi]-propyl}-ester,
1,4-Dihydro-2,6-dimethyl-4-(2-difluormethoxyphenyl)-pyridin-3,5-dicarbonsäure-3-ethyl-5-{3-[3-(4,4-diphenylpiperidyl-1)-propoxi]-propyl}-ester,
1,4-Dihydro-2,6-dimethyl-4-(3-nitrophenyl)-pyridin-3,5-dicarbonsäure-3-methyl-5-{3-[3-(4,4-dihydroxyphenylpiperidyl-1)-propoxi]-propyl}-ester,
4-(2,3-Dichlorphenyl)-1,4-dihydro-2,6-dimethylpyridin-3,5-dicarbonsäure-3-methyl-5-{3-[3-(4,4-diphenylpiperidyl-1)-propoxi]-propyl}-ester,
4-(2,1,3-Benzoxdiazol-4-yl)-1,4-dihydro-2,6-dimethylpyridin-3,5-dicarbonsäure-3-methyl-5-{3-[3-(4,4-diphenylpiperidyl-1)-propoxi]-propyl}-ester,
4-(3-Cyanphenyl)-1,4-dihydro-2,6-dimethylpyridin-3,5-dicarbonsäure-3-methyl-5-{3-[3-(4,4-diphenylpiperidyl-1)-propoxi]-propyl}-ester,
1,4-Dihydro-2,6-dimethyl-4-(2-methoxyphenyl)-pyridin-3,5-dicarbonsäure-3-methyl-5-{3-[3-(4,4-diphenylpiperidyl-1)-propoxi]-propyl}-ester,
1,4-Dihydro-2,6-dimethyl-4-(2-pyridyl)-pyridin-3,5-dicarbonsäure-3-methyl-5-{3-[3-(4,4-diphenylpiperidyl-1)-propoxi]-propyl}-ester und
1,4-Dihydro-2,6-dimethyl-4-(5-methyl-2-thienyl)-pyridin-3,5-dicarbonsäure-3-methyl-5-{3-[3-(4,4-diphenylpiperidyl-1)-propoxi]-propyl}-ester,
und ihre Salze.

Die Verbindungen der Formel I besitzen an der 4-Position im 1,4-Dihydropyridin ein Chiralitätszentrum. Die Erfindung umfaßt daher sowohl die Enantiomeren und bei Vorliegen eines weiteren Chiralitätszentrums die Diastereomeren, als auch deren Gemische und Racemate. Besonders bevorzugt sind in diesem Zusammenhang die Enantiomeren, die in der 4-Position im Dihydropyridin die gleiche Konfiguration aufweisen wie die Enantiomeren (-)-2-Amino-1,4-dihydro-6-methyl-4-(3-nitrophenyl)-pyridin-3,5-dicarbonsäure-3-ethyl-5-[3-(4,4-diphenylpiperidyl-1)-propyl]-ester bzw. (-)-1,4-Dihydro-2,6-dimethyl-4-(3-nitrophenyl)-pyridin-3,5-dicarbonsäure-3-methyl-5-{2-[2-(4,4-diphenylpiperidyl-1)-ethoxi]-ethyl}-ester-hydrochloride, die das linear polarisierte Licht der Wellenlänge 589 nm mit $[\alpha]^{22}_{D}$ = - 0,9° bzw. mit $[\alpha]^{22}_{D}$ = - 56,1° (c = 1, Methanol) drehen.

Ein weiterer Gegenstand der Erfindung ist ein Verfahren zur Herstellung der erfindungsgemäßen Verbindungen und ihrer Salze. Das Verfahren ist dadurch gekennzeichnet, daß man

1. zur Herstellung der Verbindungen der Ausgestaltung a Amidine der Formel IIa

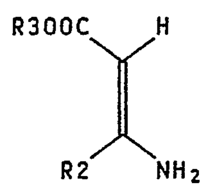

(IIa),

mit Benzylidencarbonsäurederivaten der Formel IIIa

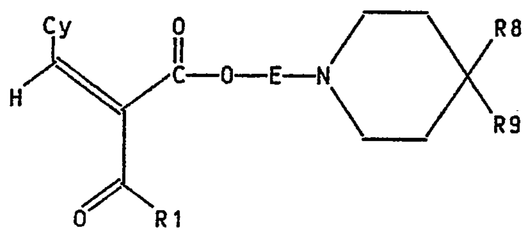

(IIIa),

als solche(n) oder in Form ihrer Salze umsetzt und gewünschtenfalls anschließend erhaltene Salze in die freien Basen oder erhaltene Basen in die Salze überführt, wobei Cy, E, R1, R2, R3, R4, R5, R8 und R9 die

für die Ausgestaltung a angegebenen Bedeutungen haben.

Das Verfahren ist weiterhin dadurch gekennzeichnet, daß man

2. zur Herstellung der Verbindungen der Ausgestaltung b

a) Zimtsäurederivate der Formel IIb

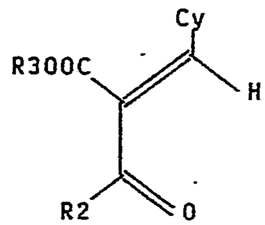

(IIb),

mit Enaminderivaten der Formel IIIb

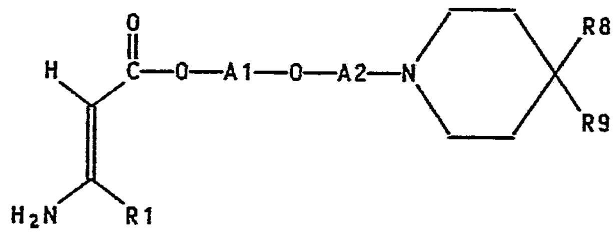

(IIIb),

oder

b) Zimtsäurederivate der Formel IIb mit Ammoniak und ß-Ketocarbonsäurederivaten der Formel IV

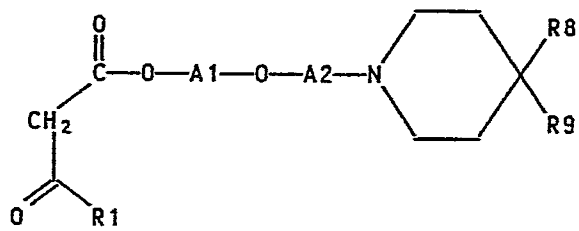

(IV),

oder

c) Enamine der Formel V

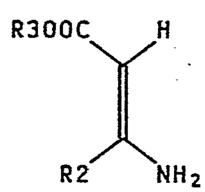

(V),

mit Benzylidencarbonsäurederivaten der Formel VI

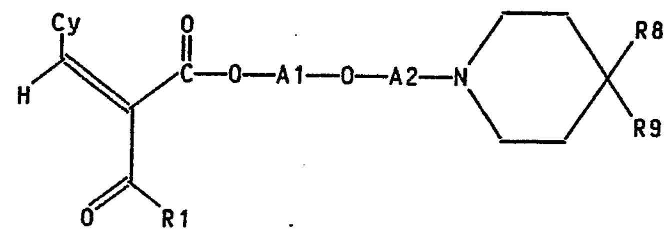

(VI),

oder

d) Ketoverbindungen der Formel VII

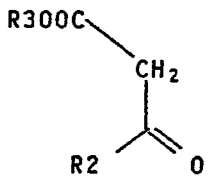

(VII),

mit Ammoniak und Benzylidencarbonsäurederivaten der Formal VI, oder

e) Aldehyde der Formel VIII

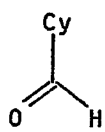

(VIII),

mit Enaminen der Formel V und ß-Ketocarbonsäurederivaten der Formel IV, oder

f) Aldehyde der Formel VIII mit Enaminderivaten der Formel IIIb und Kotoverbindungen der Formel VII, oder

g) 1,4-Dihydropyridine der Formel IX

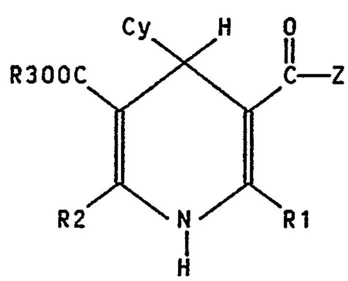

(IX),

mit Aminderivaten der Formel X

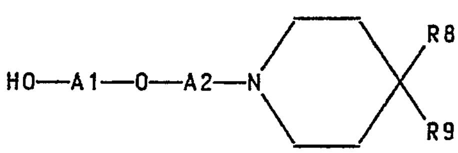

(X),

oder

h) 1,4-Dihydropyridinderivate der Formel XI

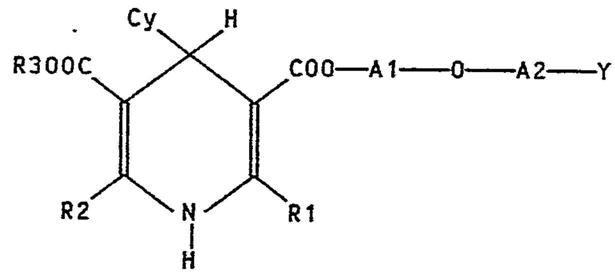

(XI),

mit Aminen der Formel XII

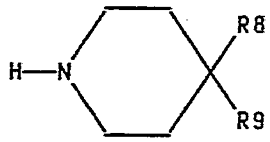

(XII),

als solche(n) oder in Form ihrer Salze umsetzt und gewünschtenfalls anschließend erhaltene Salze in die freien Basen oder erhaltene Basen in die Salze überführt, wobei Cy, A1, A2, R1, R2, R3, R4, R5, R8 und R9 die für die Ausgestaltung b angegebenen Bedeutungen haben, Z gemeinsam mit der Carbonylgruppe, woran es gebunden ist, eine Carboxylgruppe oder ein reaktives Carbonsäurederivat (z. B. ein Carbonsäurehalogenid) und Y eine Fluchtgruppe darstellt.

Ausgestaltung des Verfahrens sind solche, bei denen in den Formeln IIa, IIb, IIIa, IIIb und IV bis XII die Substituenten bzw. Symbole Cy, E, A1, A2, R1, R2, R3, R4, R5, R8 und R9, die in den Unter-und Nebenansprüchen angegebenen Bedeutungen haben, Z gemeinsam mit der Carbonylgruppe, woran es gebunden ist, eine Carboxylgruppe oder ein reaktives Carbonsäurederivat und Y eine Fluchtgruppe darstellt.

Das Verfahren gemäß 1. und 2. wird in geeigneten, vorzugsweise inerten organischen Lösungsmitteln durchgeführt. Beispielsweise seien genannt Alkohole, wie Ethanol, Methanol, Isopropanol oder insbesondere t-Butanol, Kohlenwasserstoffe, wie Toluol oder Xylol, Ether, wie Dioxan, Diethylether, Tetrahydrofuran, Glykolmonoethylether, Glykoldimethylether oder sonstige, beispielsweise polare Lösungsmittel wie Dimethylformamid, Dimethylsulfoxid, Acetonitril oder Hexamethylphosphorsäuretriamid, oder chlorierte Kohlenwasserstoffe wie Methylenchlorid, Chloroform oder Tetrachlorethylen.

Die Reaktionstemperaturen können - je nach Reaktivität der Edukte - in einem weiten Bereich variieren. Im allgemeinen wird die Umsetzung bei Temperaturen zwischen 20°C und 150°C, vorzugsweise zwischen 20°C und 100°C, insbesondere bei der Siedetemperatur des verwendeten Lösungsmittels durchgeführt.

Die Durchführung des erfindungsgemäßen Verfahrens gemäß 1. erfolgt in Gegenwart eines basischen Kondensationsmittels, beispielsweise in Gegenwart eines Alkalialkoholates, wie Natriummethylat oder Natriumethylat.

Das Verfahren gemäß 2. kann bei Normaldruck oder bei erhöhtem Druck durchgeführt werden, wobei das Arbeiten bei Normaldruck die Regel ist und erhöhter Druck insbesondere bei Umsetzungen mit Ammoniak zur Anwendung kommen kann.

Bei der Durchführung des erfindungsgemäßen Verfahrens gemäß Varianten a bis f werden die an der Reaktion beteiligten Stoffe in der Regel jeweils in molaren Mengen eingesetzt, wobei jedoch - je nach Reaktionsbedingung - gewünschtenfalls auch ein Überschuß (beispielsweise an Ammoniak bei den Varianten b und d) eingesetzt werden kann.

Bei der Durchführung des Verfahrens gemäß Variante g kommen ähnliche Reaktionsbedingungen wie bei den Varianten a bis f zur Anwendung, jedoch sind - je nach Art des Substituenten Z - gegebenenfalls zusätzliche Maßnahmen erforderlich. Stellt Z beispielsweise eine Hydroxylgruppe dar, so ist die Reaktion bevorzugt in Gegenwart eines wasserabspaltenden oder wasserbindenden Kondensationsmittels (wie z.B. Dicyclohexylcarbodiimid) durchzuführen. Stellt Z ein Halogenatom (z. B. ein Chloratom) dar, so ist die Reaktion gewünschtenfalls in Gegenwart einer Base (z. B. eines tertiären organischen Amins, wie Triethylamin, oder eines anorganischen Carbonates, wie Natriumcarbonat) durchzuführen.

Bei der Durchführung des Verfahrens gemäß Variante h kommen ähnliche Reaktionsbedingungen wie bei den Varianten a bis f zur Anwendung. Die Umsetzung erfolgt in einer Weise, wie sie für die Herstellung von sekundären bzw. tertiären Aminen bekannt ist. Je nach Art der Fluchtgruppe Y, die vorzugsweise ein Halogenatom, insbesondere ein Chlor-oder Bromatom ist, kann die Reaktion gewünschtenfalls in Gegenwart einer Base (z.B. eines anorganischen Carbonates, wie Kaliumcarbonat) oder unter Einsetzung eines Überschusses von Amin XII durchgeführt werden.

Die enantiomer reinen Verbindungen und ihre Salze, die ebenfalls Gegenstand der Erfindung sind, erhält man beispielsweise, indem man die nach dem oben beschriebenen Verfahren erhaltenen Racemate mit einer enantiomer reinen optisch aktiven Säure umsetzt, die erhaltenen diastereomeren Salze trennt, aus den gewünschten diastereomeren Salze die Enantiomeren durch Zugabe von Base freisetzt und sie gewünschtenfalls anschließend in ihre Salze überführt.

Als optisch aktive Säuren seien beispielsweise die Di-0,0'-p-toluoylweinsäure oder insbesondere die Di-0,0'-benzoylweinsäure genannt. Als Trennverfahren eignet sich vorzugsweise die Umkristallisation.

Die mit Hilfe dieser Methoden getrennten, konfigurativ einheitlichen diastereomeren Salze werden durch Zugabe vorzugsweise anorganischer Basen, wie, z.B. Ammoniak, oder mit Hilfe von basischen Ionenaustauschern in die optisch aktiven, enantiomer reinen erfindungsgemäßen Verbindungen überführt.

Dieses Verfahren zur Herstellung enantiomer reiner Verbindungen wird vorzugsweise für die Herstellung der enantiomer reinen Dihydropyridine der Ausgestaltung a eingesetzt.

Alternativ erhält man die enantiomer reinen erfindungsgemäßen Verbindungen, indem man von enantiomer reinen Zwischenprodukten ausgeht. Hier sind insbesondere die enantiomer reinen, 1,4-Dihydropyridine der Formel IX zu nennen, aus denen man durch Umsetzung mit den Aminderivaten X - wie bei Verfahrensvariante g beschrieben - die gewünschten enantiomer reinen erfindungsgemäßen Endprodukte erhält. Dieser Weg wird vorzugsweise zur Herstellung der enantiomer reinen Dihydropyridine der Ausgestaltung b eingeschlagen.

Die Isolierung und Reinigung der nach 1. oder 2. erhaltenen erfindungsgemäßen Substanzen erfolgt in an sich bekannter Weise z. B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen.

Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die Ausgangsverbindungen sind literaturbekannt oder können in Analogie zu literaturbekannten Methoden hergestellt werden. Die Benzylidencarbonsäurederivate IIIa können beispielsweise in Analogie zu G. Jones ["The Knoevenagel Condensation" in Org. Reactions, Vol. XV, 204f (1967)] hergestellt werden. Die Amidine IIa können nach H. Yamanaka et al., Heterocycles (1976), 1854 hergestellt werden. Die Zimtsäurederivate IIb und die Benzylidencarbonsäurederivate VI können beispielsweise in Analogie zu G. Jones ["The Knoevenagel Condensation" in Org. Reactions, vol. XV, 204f (1967)] hergestellt werden. Die Enaminderivate IIIb bzw. die Enamine V sind bei spielsweise analog A.C. Cope [J. Amer. Chem. Soc. 67, 1017 (1945)] erhältlich. β-Ketocarbonsäurederivate IV und Ketoverbindungen VII können gemäß D. Borrmann ["Umsetzung von Diketen mit Alkoholen, Phenolen und Mercaptanen" in Houben-Weyl, Methoden der Organischen Chemie, Vol. VII/4, 230ff (1968)] oder Y. Oikawa et al. [J. Org. Chem. 43, 2087 (1978)] hergestellt werden. Die Verbindungen IX sind aus entsprechenden Ausgangsverbindungen analog Verfahrensvariante a bis f zugänglich. Die zur Herstellung enantiomer reiner Verbindungen benötigten enantiomer reinen 1,4-Dihydropyridine IX sind aus Chem. Pharm. Bull. 28 , 2809 (1980) bekannt oder analog dazu erhältlich. Verbindungen X sind durch Umsetzung entsprechender Piperidine mit omega-Halogenalkanolen erhältlich. Die Dihydropyridinderivate XI erhält man durch Umsetzung von Enaminen der Formel V mit z.B. entsprechend substituierten omega-Halogen-2-acyl-acrylsäureestern, die ihrerseits wiederum aus Aldehyden der Formel VIII und geeigneten beta-Keto-omega-halogencarbonsäureestern zugänglich sind.

Das vorstehende Herstellungsverfahren ist lediglich zur Verdeutlichung angegeben, und die Herstellung der erfindungsgemäßen Verbindungen der Formel I ist nicht auf dieses Verfahren beschränkt. Vielmehr ist auch jede Modifikation dieses Verfahrens in gleicher Weise für die Herstellung der erfindungsgemäßen Verbindungen anwendbar.

Die folgenden Herstellungsbeispiele sollen die Erfindung näher erläutern, ohne sie einzuschränken. Schmp. bedeutet Schmelzpunkt, h steht für Stunden, Kp. steht für Siedepunkt, Zers. bedeutet Zersetzung.

Beispiele

Endprodukte

1. 1,4-Dihydro-2,6-dimethyl-4-(3-nitrophenyl)-pyridin-3,5-dicarbonsäure-3-methyl-5-{2-[2-(4,4-diphenylpiperidyl-1)-ethoxi]-ethyl}-ester-hydrochlorid

5,4 g 2-Acetyl-3-(3-nitrophenyl)-acrylsäure-2-[2-(4,4-diphenylpiperidyl-1)-ethoxi]-ethylester und 1,2 g 3-Aminocrotonsäuremethylester werden in 50 ml 2-Propanol und 0,5 ml Essigsäure 5 h unter Rückfluß zum Sieden erhitzt. Die abgekühlte Lösung wird zur Trockene eingeengt, der fest aufgeschäumte Rückstand in wenig Dichlormethan gelöst und die Lösung über 3 $\times$ 20 cm Kieselgel chromatographiert (Eluens: Dichlormethan/Methanol/Essigsäure 9 + 1 + 1). Die Produkfraktion wird eingeengt, der Rückstand in Dichlormethan aufgenommen und mit etherischer Salzsäure versetzt. Nach dem erneuten Einengen der Produktlösung wird der Rückstand in ca. 10 ml Dichlormethan gelöst und die Titelverbindung durch langsames Eintropfen in 500 ml einer gut gerührten Mischung aus gleichen Teilen Diethylether und Petrolether amorph ausgefällt. Schmp.: 124-138°C, Ausbeute: 4,9 g.

2. (-)-1,4-Dihydro-2,6-dimethyl-4-(3-nitrophenyl)-pyridin-3,5-dicarbonsäure-3-methyl-5-{2-[2-(4,4-diphenylpiperidyl-1)-ethoxi]-ethyl}-ester-hydrochlorid

997 mg (-)-1,4-Dihydro-2,6-dimethyl-4-(3-nitrophenyl)-pyridin-3,5-dicarbonsäure-3-methylester werden mit 3 ml Oxalylchlorid versetzt. Die Mischung wird bei Raumtemperatur solange gerührt, bis keine weitere Gasentwicklung mehr erkennbar ist. Unter Zusataz von je 5 ml abs. Toluol engt man den Ansatz dreimal zur Trockene ein. Der erhaltene braune feste Rückstand wird in 3 ml abs. Dichlormethan suspendiert und die Suspension unter $N_2$-Begasung in eine auf 0°C gekühlte Lösung von 1,09 g N-[2-(2-Hydroxiethoxi)-ethyl]-4,4-diphenylpiperidin und 0,6 ml Triethylamin eingetropft. Nach dem Zutropfen rührt man die Mischung noch 2 h bei Raumtemperatur und engt dann zur Trockene ein. Der verbleibende bräunliche Rückstand wird in 100 ml Dichlormethan aufgenommen und dreimal mit je 50 ml Wasser extra hiert. Nach dem Trocknen der organischen Phase über Natriumsulfat engt man die bräunliche klare Lösung weitgehend ein und chromatographiert den öligen Rückstand über eine 2 $\times$ 30 cm Kieselgelsäule mit Dichlormethan/Ethanol (98 + 2) als Elutionsmittel. Nach dem Einengen der chromatographisch einheitlichen Produktfraktion nimmt man den verbleibenden gelblichen Rückstand in 5 ml Dichlormethan auf und versetzt die Lösung mit etherischer Salzsäure. Nach erneutem Einengen der Hydrochloridlösung zur Trockene wird der fest aufgeschäumte Rückstand in 3 ml Dichlormethan gelöst und das Produkt durch Eintropfen der Lösung in 1 l Petrolether/Diethylether (2 + 1) amorph ausgefällt. Nach Absaugen und Trocknen des Niederschlages erhält man die Titelverbindung als feines graues Pulver vom Schmp.: 118-128°C (langsames Zerfließen); $[\alpha]_D^{21}$ = - 0.9° (c = 1, Methanol); Ausbeute: 490 mg.

3. 1,4-Dihydro-2,6-dimethyl-4-(3-nitrophenyl)-pyridin-3,5-dicarbonsäure-3-methyl-5-{2-[2-(2-(4,4-diphenylpiperidyl-1)-ethoxi)-ethoxi]-ethyl}-ester-hydrochlorid-hydrat

Aus 4,54 g Acetessigsäure-{2-[2-(2-(4,4-diphenylpiperidyl-1)-ethoxi)-ethoxi]-ethyl}-ester, 1,41 g 3-Nitrobenzaldehyd und 1,20 g 3-Aminocrotonsäure in 80 ml 2-Propanol und 0,5 ml Eisessig erhält man nach analoger Umsetzung der Ausgangsverbindungen und Aufarbeitung des Reaktionsgemisches wie in Beispiel 1 beschreiben die Titelverbindung als amorphes Pulver vom Schmp.: 106-115°C (langsames Zerfließen); Ausbeute: 2,5 g.

4. 2-Amino-1,4-dihydro-6-methyl-4-(3-nitrophenyl)-pyridin-3,5-dicarbonsäure-3-ethyl-5-[3-(4,4-diphenylpiperidyl-1)-propyl]-ester

Zu einer Lösung von 5,49 g 2-Acetyl-3-(3-nitrophenyl)-acrylsäure-[3-(4,4-diphenylpiperidyl-1)-propyl]-ester-hydrochlorid und 1,67 g Amidinoessigsäureethylester-hydrochlorid in 15 ml Ethanol tropft man in der Siedehitze innerhalb von 60 Min. eine aus 0,46 g Natrium und 20 ml Ethanol bereitete Natriumethylatlösung zu und kocht anschließend noch ca. 30 Min. unter Rückfluß. Nach dem Einengen des Reaktionsansatzes wird der erhaltene Rückstand zwischen Essigsäureethylester, Natriumhydrogencarbonatlösung und anschließend Wasser verteilt. Die organische Phase engt man nach dem Trocknen über Natriumsulfat ein und chromatographiert den Rückstand über eine 3 × 30 cm Kieselgelsäule mit Dichlormethan/Ethanol (95+5) als Eluens. Die Produktfraktion wird eingeengt und der fest aufgeschäumte Rückstand in wenig Methanol aufgenommen. Nach Zusatz von Diethylether/Petrolether (1+1) bis zur ersten bleibenden schwachen Trübung läßt man die Mischung im Kühlschrank 24 h stehen. Die Titelverbindung kristallisiert in feinen Plättchen vom Schmp. 174-175°C aus. Ausbeute: 4,4 g.

5. (-)-2-Amino-1,4-dihydro-6-methyl-4-(3-nitrophenyl)-pyridin-3,5-dicarbonsäure-3-ethyl-5-[3-(4,4-diphenylpiperidyl-1)-propyl]-ester

10 g des Racemates aus Beispiel 4 und 6,02 g D-(+)-0,0'-Dibenzoylweinsäurehydrat werden in 300 ml Chloroform in der Siedehitze gelöst. Nach Zusatz von 50 ml Essigsäureethylester läßt man die Lösung langsam erkalten. Man erhält ein erstes Kristallisat (10 g), das nach dem Absaugen in der Siedehitze in einem Gemisch aus Chloroform/Methanol (4 + 1) umkristallisiert wird. Das nach dem Erkalten erhaltene zweite Kristallisat (8,5 g) wird erneut in der vorstehenden Löstemittelmischung umkristallisiert. Man erhält das 0,0'-Dibenzoyltartrat der Titelverbindung als grobe gelbliche Nadeln vom Schmp.: 178-179°C (Zersetzung); $[\alpha]_D^{22}$ = + 10,3° (c = 1, Methanol); Ausbeute: 4,1 g. Das Salz wird in 300 ml Dichlormethan gelöst und die Lösung mit 200 ml konzentrierter Ammoniaklösung und anschließend mit je 100 ml Wasser dreimal extrahiert. Nach dem Trocknen der organischen Phase über Natriumsulfat engt man ein. Der verbleibende feste Rückstand wird in 5 ml Dichlormethan gelöst und das Produkt durch Eintropfen der Lösung in 1 l Petrolether amorph ausgefällt. Man erhält die Titelverbindung nach dem Absaugen als gelbliches feines Pulver vom Schmp.: 96-104°C (langsames Zerfließen); $[\alpha]_D^{22}$ = - 56,1° (c = 1, Methanol); Ausbeute: 2,31 g.

6. 2-Amino-1,4-dihydro-6-methyl-4-(3-nitrophenyl)-pyridin-3,5-dicarbonsäure-3-ethyl-5-[5-(4,4-diphenylpiperidyl-1)-pentyl]-ester

Analog Beispiel 4 erhält man die Titelverbindung aus 3,60 g Acetyl-3-(3-nitrophenyl)-acrylsäure-[5-(4,4-diphenylpiperidyl-1)-pentyl]-ester, 1,11 g Amidinoessigsäureethylester-hydrochlorid und 0,153 g Natrium in 17 ml abs. Ethanol nach 2 h Reaktionszeit als graues Pulver vom Schmp.: 98-120°C (langsames Zerfließen; amorph ausgefällt in Petrolether); Ausbeute: 1,97 g.

7. 2-Amino-4-(2,3-dichlorphenyl)-1,4-dihydro-6-methyl-pyridin-3,5-dicarbonsäure-3-ethyl-5-[3-(4,4-diphenylpiperidyl-1)-propyl]-ester-semifumarat

Analog Beispiel 4 erhält man die Titelverbindung aus 5,00 g 2-Acetyl-3-(2,3-dichlorphenyl)-acrylsäure-[3-(4,4-diphenylpiperidyl-1]--ester, 1,55 g Amidinoessigsäureethylester-hydrochlorid und 210 mg Natrium in 25 ml abs. Ethanol nach Überführung in das Semifumarat als harte würfelige Kristalle vom Schmp.: 191-93°C (aus Methanol/Diethylether); Ausbeute: 5,05 g.

8. 2-Amino-4-(4-benzo[c][1.2.5]oxdiazolyl)-1,4-dihydro-6-methyl-pyridin-3,5-dicarbonsäure-3-ethyl-5-[3-(4,4-diphenylpiperidyl-1)-propyl]-ester

Analog Beispiel 4 erhält man die Titelverbindung aus 6,11 g 2-Acetyl-3-(4-benzo[c][1.2.5]oxdiazolyl)-acrylsäure-[3-(4,4-diphenylpiperidyl-1)-propyl]-ester, 2,01 g Amidinoessigsäueethylester-hydrochlorid und 276 mg Natrium in 35 ml abs. Ethanol als feine gelbliche Kristallplättchen vom Schmp.: 127-131°C (langsames Zerfließen, aus Methanol/Diethylether); Ausbeute: 2,7 g.

9. 2-Amino-4-(2-chlorphenyl)-1,4-dihydro-6-methyl-pyridin-3,5-dicarbonsäure-3-ethyl-5-[3-(4,4-diphenylpiperidyl-1)-propyl]-ester

Analog Beispiel 4 erhält man die Titelverbindung aus 4,02 g 2-Acetyl-3-(2-chlorphenyl)-acrylsäure-[3-(4,4-diphenylpiperidyl-1)-propyl]-ester, 1,33 g Amidinoessigsäueethylester-hydrochlorid und 184 mg Natrium in 50 ml abs. Ethanol als feine gelbliche Kristallplättchen vom Schmp.: 125-128°C (aus Methanol/Diethylether); Ausbeute: 2,71 g.

10. 2-Amino-1,4-dihydro-6-methyl-4-(2-trifluormethylphenyl)-pyridin-3,5-dicarbonsäure-3-ethyl-5-[3-(4,4-diphenylpiperidyl-1)-propyl]-ester

Analog Beispiel 4 erhält man die Titelverbindung aus 4,28 g 2-Acetyl-3-(2-trifluormethylphenyl)-acrylsäure-[3-(4,4-diphenylpiperidyl-1)-propyl]-ester, 1,33 g Amidinoessigsäureethylester-hydrochlorid und 184 mg Natrium in 40 ml abs. Ethanol als feine gelbliche Kristallplättchen vom Schmp.: 115-117°C (aus Methanol/Diethylether), Ausbeute: 3,32 g.

11. 2-Amino-1,4-dihydro-6-methyl-4-(3-nitrophenyl)-pyridin-3,5-dicarbonsäure-3-methyl-5-[2-(4,4-diphenylpiperidyl-1)-ethyl]-ester

Analog Beispiel 4 erhält man die Titelverbindung aus 4,98 g 2-Acetyl-3-(3-nitrophenyl)-acrylsäure-[2-(4,4-diphenylpiperidyl-1)-ethyl]-ester, 1,52 g Amidinoessigsäuremethylester-hydrochlorid und 230 mg Natrium in 40 ml abs. Methanol als feines gelbliches Pulver vom Schmp.: 110-116°C, langsames Zerfließen (ausgefällt in Petrolether/Diethylether (2 + 1); Ausbeute: 3,12 g.

Ausgangsverbindungen

A. 2-Acetyl-3-(3-nitrophenyl)-acrylsäure-2-[2-(4,4-diphenylpiperidyl-1)-ethoxi]-ethylester

4,1 g Acetessigsäure-2-[2-(4,4-diphenylpiperidyl-1)-ethoxi]-ethylester, 15 g 3-Nitrobenzaldehyd, 8 ml Essigsäure und 0,5 ml Piperidin werden in 300 ml Toluol am Wasserabscheider zum Sieden erhitzt. Nach Abscheiden von 1,9 ml Wasser wird die abgekühlte Lösung mit gesättigter Natriumhydrogencarbonatlösung und anschließend mit Wasser gewaschen. Nach dem Trocknen der organischen Phase mit Natriumsulfat engt man die klare rötlich-braune Lösung im Hochvakuum ein. Der erhaltene zäh-viskose Rückstand wird ohne weitere Reinigung zur Kondensation eingesetzt. Ausbeute: 53 g Rohprodukt als cis/trans-Isomerengemisch. Als Schlepper sind ferner geeignet: Benzol, chlorierte Kohlenwasserstoffe. Die Ausbeute an Rohprodukt beträgt 95-100% der Theorie.

B. Acetessigsäure-2-[2-(4,4-diphenylpiperidyl-1)-ethoxi]-ethylester

Zu 32,5 g N-[2-(2-Hydroxiethoxi)-ethyl]-4,4-diphenylpiperidin und ca. 0,1 g N,N-Dimethylaminopyridin in 200 ml einer 50%igen Diketenlösung in Aceton. Nach 1 h Sieden unter Rückfluß läßt man die Lösung erkalten und engt im Hochvakuum bis zur Gewichtskonstanz ein. Das verbleibende hellgelbe, zähe Öl wird ohne weitere Reinigung für die nächste Stufe eingesetzt.

In analoger Weise wird der Acetessigsäure-{2-[2-(2-(4,4diphenylpiperidyl-1)-ethoxi)-ethoxi]-ethyl}ester - ausgehend von Triethylenglycolmonochlorhydrin und durch vorherige Umsetzung wie bei C beschrieben - hergestellt.

C. N-[2-(2-Hydroxiethoxi)-ethyl]-4,4-diphenylpiperidin

2 g 4,4-Diphenylpiperidin, 50 g Diethylenglycolmonochlorhydrin, 250 g fein gepulvertes Kaliumcarbonat und 1 g Kaliumiodid werden in 1,2 l eines 1:1 Gemisches aus Dioxan und 1-Butanol 50 h unter Rückfluß und kräftigem Rühren zum Sieden erhitzt. Nach dem Abkühlen wird filtriert und das Filtrat eingeengt. Der ölige Rückstand wird in Essigsäureethylester aufgenommen und die Lösung nochmals filtriert. Nach dem Einengen des Filtrats bis zur Gewichtskonstanz (Hochvakuum) erhält man die Titelverbindung als wachsartigen, zähen Rückstand. Ausbeute: 106 g. Mit etherischer Salzsäure erhält man das Hydrochlorid, das in 2-Propanol umkristallisiert wird. Schmp.: 120-121 °C.

Die Herstellung weiterer Ausgangsverbindungen ist z. B. in der Europäischen Patentanmeldung 176 956 beschrieben.

Gewerbliche Anwendbarkeit

Die erfindungsgemäßen Verbindungen der Formel I und ihre Salze besitzen wertvolle Eigenschaften, die sie gewerblich verwertbar machen. Sie stellen insbesondere wirksame Vasodilatoren mit coronartherapeutischen Eigenschaften dar. Die pharmakologische Wirksamkeit der erfindungsgemäßen Verbindungen zeigt sich insbesondere in einer langsam eintretenden, starken und optimal anhaltenden Blutdrucksenkung. Darüberhinaus besitzen die erfindungsgemäßen Verbindungen hemmende Wirkung auf den Calciumeinstrom sowie fördernde Wirkung auf den Kaliumausstrom von Zellen, glattmuskulär relaxierende und peripher, coronar, cerebral und renal gefäßerweiternde sowie salidiuretische, antithrombotische, antiarteriosklerotische und günstige hämorheologische Eigenschaften.

In ihrer ausgezeichneten Wirksamkeit, die gepaart ist mit einer geringen Toxizität und dem Fehlen wesentlicher Nebenwirkungen, unterscheiden sich die erfindungsgemäßen Verbindungen in überraschender und vorteilhafter Weise von den Verbindungen des Standes der Technik.

Als vorteilhafte Eigenschaften der Verbindungen I sind beispielsweise zu nennen: das Ausmaß der Blutdrucksenkung, die gute Steuerbarkeit der Blutdrucksenkung, die - insbesondere bei den Verbindungen der Ausgestaltung a - im Vergleich zu den Verbindungen des Standes der Technik überraschend geringe Herzfrequenzsteigerung, die ausgezeichnete Bioverfügbarkeit, die große therapeutische Breite, das Fehlen zentraler Nebenwirkungen, das Fehlen kinetischer Interaktionen mit anderen Substanzen, das Ausbleiben einer Toleranzentwicklung, die ausgewogenen physikalischen Eigenschaften und die große Stabilität.

Die ausgezeichnete Wirksamkeit der erfindungsgemäßen Verbindungen der Formel I und ihrer Salze gestattet ihren Einsatz in der Humanmedizin, wobei als Indikation insbesondere primäre (essentielle) und sekundäre, arterielle und pulmonale Hypertonien aller Schweregrade, koronare Herzkrankheiten (Koronarinsuffizienz, Angina Pectoris, Myocardinfarkt etc.), periphere und cerebrale Zirkulationsstörungen (Gehirnschlag, temporäre cerebrale Durchblutungsstörungen, Migräne, Schwindel, renale Arterienverengung etc.), hypertrophe Kardiomyopathie, Herzinsuffizienz, Krankheiten, die auf einer erhöhten Wasser-und Natriumretention beruhen und Krankheiten, die auf einem erhöhten Calciumeinstrom beruhen, wie z.B. Spasmen glattmuskulärer Organe (Atemwege, Gastrointestinaltrakt, Urogenitaltrakt etc.) sowie Arrhythmie, Arteriosklerose und Zellschädigungen verschiedener Genese (z. B. Hypoxie) in Betracht kommen.

Ein weiterer Gegenstand der Erfindung ist daher ein Verfahren zur Behandlung von Säugetieren, insbesondere Menschen, die an einer der obengenannten Krankheiten erkrankt sind. Das Verfahren ist dadurch gekennzeichnet, daß man dem erkrankten Individuum eine therapeutisch wirksame und pharmakologisch verträgliche Menge einer oder mehrerer Verbindungen der Formel I verabreicht.

Gegenstand der Erfindung sind außerdem die Verbindungen der Formel I zur Anwendung bei der Behandlung der genannten Krankheiten.

Ebenso umfaßt die Erfindung die Verwendung von Verbindungen der Formel I bei der Herstellung von Arzneimitteln, die zur Bekämpfung der genannten Krankheiten eingesetzt werden.

Ein weiterer Gegenstand der Erfindung sind Arzneimittel, die eine oder mehrere Verbindungen der allgemeinen Formel I enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfsstoffen in Form von Tabletten, Dragees, Kapseln, Suppositorien, Pflastern (z.B. als TTS), Emulsionen, Suspensionen, Aerosolen, Sprays, Salben, Cremes, Gelen oder Lösungen eingesetzt, wobei der Wirkstoffgehalt vorteilhafterweise zwischen 0,1 und 95% beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten, Hilfsstoffen und anderen Wirkstoffträgern können beispielsweise Antioxidantien, Dispergiermittel, Emulgatoren, Entschäumer, Geschmackskorrigentien, Konservierungs-Die Wirkstoffe können oral, rektal, per inhalationem oder parenteral (insbesondere perlingual, intravenös oder percutan) appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 10, vorzugsweise 0,05 bis 5 mg/kg Körpergewicht, gewünschtenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Bei einschleichender Dosierung wird zu Beginn der Behandlung eine geringere Dosis verabreicht, dann langsam auf ein höhere Dosis übergegangen. Nach Erreichen des gewünschten Therapieerfolges wird wieder auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der genannten Krankheiten eingesetzt werden, so können die pharmazeutischen Zubereitungen auch einen oder mehrere andere pharmakologisch aktive Bestandteile anderer Arzneimittelgruppen, wie andere Vasodilatoren, Antihypertensiva, alpha-1-Rezeptorenblocker, alpha-2-Rezeptorstimulatoren, beta-1-Rezeptorenblocker, beta-2-Rezeptorstimulatoren, ACE-Hemmstoffe, Nitroverbindungen, Cardiotonika, Diuretika, Saluretika, Alkaloide, Analgetika, Lipidsenker, Antikoagulantien, Anticholinergika, Methylxanthine, Antiarrhythmika, Antihistaminika, Dopaminstimulatoren, Serotonin-Rezeptorenblocker etc., wie Nifedipin, Dihydralazin, Prazosin, Clonidin, Atenolol, Labetalol, Fenoterol, Captopril, Isosorbiddinitrat, Digoxin, Milrinon, Mefrusid, Clopamid, Spironolacton, Chlorthalidon,, Furosemid, Polythiazid, Hydrochlorothiazid, Reserpin, Dihydroergocristin, Rescinnamin, Rauwolfia-Gesamtalkaloide, Acetylsalicylsäure, Bezafibrat, Warfarin, Atropin, Theophyllin, Lidocain, Astemizol, Bromocryptin, Ketanserin etc. enthalten.

## Pharmakologie

Die antihypertensive Wirksamkeit der erfindungsgemäßen Verbindungen kann am Modell der spontan hypertonen Ratte nachgewiesen werden.

Zur Bestimmung der antihypertensiven Wirkung werden die unten aufgeführten Verbindungen in den angegebenen Dosen an vier aufeinander folgenden Tagen an je 6 männlichen Ratten (Stamm SHR/N/Ibm/Bm, 250-350 g) mit genetisch bedingtem Hochdruck (systolischer Blutdruck > 180 mmHg) täglich einmal mittels Schlundsonde verabfolgt. Die Messung des Blutdrucks erfolgt jeweils 6 und gegebenenfalls 2 oder 24 Stunden nach Substanzgabe.

Die Blutdruckmessung wird in einer Wärmekammer bei 36°C vorgenommen, um eine bessere Durchblutung der Schwanzarterie zu erreichen. Hierzu werden die Tiere in perforierte Lochblechkäfige verbracht und 20 - 40 Min. nach Beginn der Aufwärmung gemessen. Zur Messung des systolischen arteriellen Drucks wird eine ringförmige Manschette mit aufblasbarer Gummimembran zur Unterbindung der Durchblutung und ein ringförmiger Piezokristallaufnehmer zur Erfassung der Pulswellen auf den Schwanz aufgeschoben. Nach erfolgter Unterbindung des Blutstroms in der Schwanzarterie wird der Manschettendruck kontinuierlich reduziert. Die Wiederkehr der Pulswellen bei Druckablassen wird automatisch als systolischer Blutdruck erkannt und ausgedruckt (Bühler, R. et al.: Microprocessor-based automation of blood pressure measurement in the conscious rat. Proceedings of the 4th international symposium on rats with spontaneous hypertension and related studies, Rascher, R. et al. (Eds.), Schattauer Verlag, Stuttgart, New York, 1982, S. 410-413). Pulssignale und Druckverlauf werden zur Auswertung graphisch aufgezeichnet.

Zur Gewöhnung an den Meßvorgang werden die Tiere vor Substanzprüfung 14 Tage trainiert. In der zweiten Trainingswoche werden Blutdruck-Vorwerte erhoben. Tiergruppen, die Substanz erhalten, werden gegen eine Kontrollgruppe geprüft.

In der anschließenden Tabelle werden die untersuchten Verbindungen durch laufende Nummern gekennzeichnet, die mit den jeweiligen Beispielnummern übereinstimmen.

Tabelle I gibt für die Vertreter der erfindungsgemäßen Verbindungen die prozentuale Senkung des Blutdrucks (BP) nach oraler Verabreichung bei der Ratte wieder.

Tabelle I

%-Änderungen (BP) an genetisch hypertonen Ratten nach täglich einmaliger p.o.-Applikation an vier aufeinanderfolgenden Tagen (N=6/Dosis).

| lfd. Nr. | Dosis µmol/kg | BP (% Änderung vs. Kontrolle), Mittelwert für Meßzeitpunkte: Stunden nach Gabe (Tage) | | |
|---|---|---|---|---|
| | | 2h (1.+4.Tag) | 6h (1.-4.Tag) | 24h (1.+3.Tag) |
| 1 | 25 | -49,0 | -30,0 | - 4,0 |
| 2 | 25 | -45,5 | -34,0 | - 5,0 |
| 4 | 25 | -53,5 | -49,0 | -33,5 |
| 6 | 25 | -20,0 | -30,3 | - 4,5 |
| 7 | 25 | -10,0 | -29,5 | - 3,5 |

**Ansprüche**

1. Verbindungen der Formel I

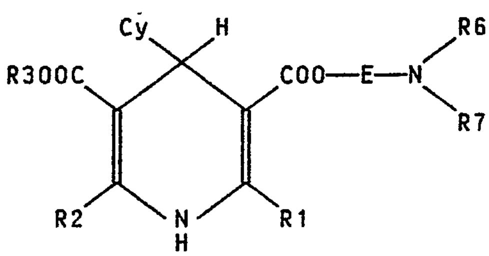

(I)

worin Cy einen Cyclus der Formel

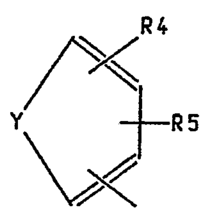

darstellt, in dem Y Sauerstoff (O), Schwefel (S), Vinylen (-CH=CH-), Azomethin (-CH=N-) oder eine Gruppe der Formel

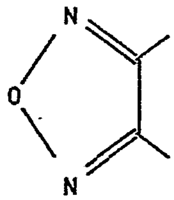

oder

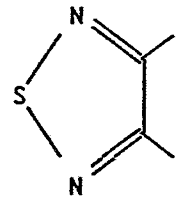

bedeutet,
R1 Wasserstoff, 1-6C-Alkyl oder 3-7C-Alkoxyalkyl bedeutet,
R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxycarbonyl, 2-5C-Acyl, Amino oder Mono-oder Di-1-4C-alkylamino bedeuten,
R6 und R7 gemeinsam und unter Einschluß des Stickstoffatoms, an das beide gebunden sind, einen Rest der Formel

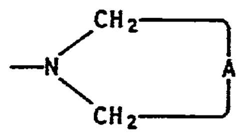

darstellen, worin
A $-CH_2-C(R8)R9-CH_2-$bedeutet,
R8 Aryl bedeutet und
R9 Aryl bedeutet, wobei
Aryl für einen Ring der Formel

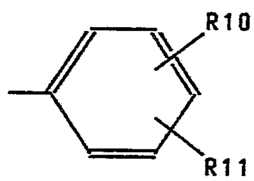

steht, in dem R10 und R11 gleich oder verschieden sind und die Bedeutung Wasserstoff (H), 1-4C-Alkyl, 1-4C-Alkoxy, Halogen, Hydroxy oder Trifluormethyl haben,
und worin
entweder
E 2-5C-Alkylen,
R2 Amino ($NH_2$) und
R3 1-6C-Alkyl oder 3-7C-Alkoxyalkyl bedeuten,
oder
E A1-O-A2,
R2 Wasserstoff, 1-6C-Alkyl oder 3-7C-Alkoxyalkyl und
R3 Wasserstoff, 1-6C-Alkyl oder 3-7C-Alkoxyalkyl bedeuten,
wobei
A1 2-4C-Alkylen und
A2 2-4C-Alkylen oder 2C-Alkylenoxy-2C-alkylen bedeutet,
und die Salze dieser Verbindungen.

2. Verbindungen der Formel Ia

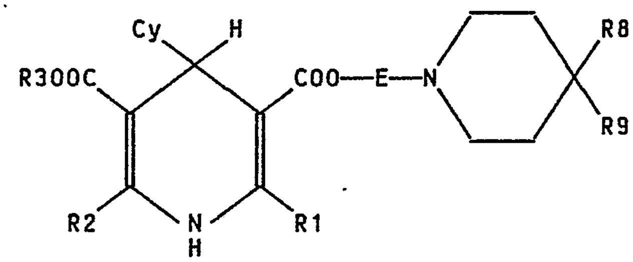

(Ia)

worin Cy einen Cyclus der Formel

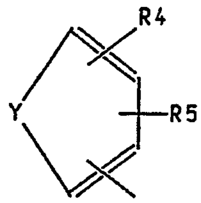

darstellt, in dem Y Sauerstoff (O), Schwefel (S), Vinylen (-CH = CH-), Azomethin (-CH = N-) oder eine Gruppe der Formel

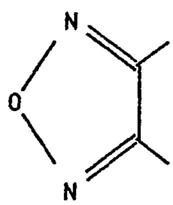

oder

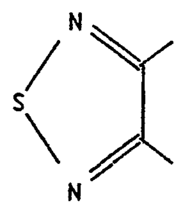

bedeutet,
E 2-5C-Alkylen bedeutet,
R1 Wasserstoff, 1-6C-Alkyl oder 3-7C-Alkoxyalkyl bedeutet,
R2 Amino ($NH_2$) bedeutet,
R3 1-6C-Alkyl oder 3-7C-Alkoxyalkyl bedeutet,
R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxycarbonyl, 2-5C-Acyl, Amino oder Mono-oder Di-1-4C-alkylamino bedeuten,
R8 Aryl bedeutet und
R9 Aryl bedeutet, wobei
Aryl für einen Ring der Formel

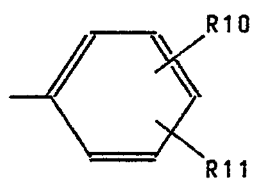

steht, worin R10 und R11 gleich oder verschieden sind und die Bedeutung Wasserstoff (H), 1-4C-Alkyl, 1-4C-Alkoxy, Halogen, Hydroxy oder Trifluormethyl haben,
und die Salze dieser Verbindungen.

3. Verbindungen der Formel Ia nach Anspruch 2, worin
Cy 3-Nitrophenyl, 2-Chlorphenyl, 2,3-Dichlorphenyl, 2-Trifluormethylphenyl oder Benzoxdiazolyl bedeutet,
E Ethylen ($-CH_2-CH_2-$), Trimethylen ($-CH_2-CH_2-CH_2-$) oder Pentamethylen ($-CH_2-CH_2-CH_2-CH_2-CH_2-$) bedeutet,
R1 Methyl bedeutet,
R2 Amino ($NH_2$) bedeutet,

R3 Methyl oder Ethyl bedeutet,
R8 Phenyl bedeutet und
R9 Phenyl bedeutet,
und ihre Salze.

4. Verbindungen der Formel Ib

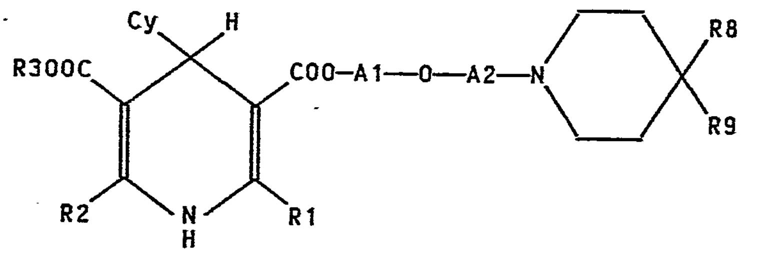

(Ib)

worin Cy einen Cyclus der Formel

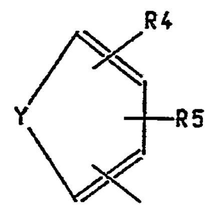

darstellt, in dem Y Sauerstoff (O), Schwefel (S), Vinylen (-CH=CH-), Azomethin (-CH=N-) oder eine Gruppe der Formel

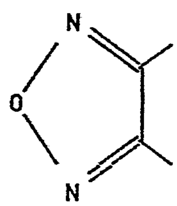

oder

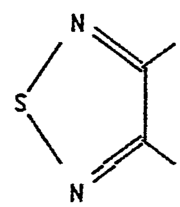

bedeutet,
A1 2-4C-Alkylen bedeutet,
A2 2-4C-Alkylen oder 2C Alkylenoxy-2C-alkylen bedeutet,
R1 und R2 gleich oder verschieden sind und Wasserstoff, 1-6C-Alkyl oder 3-7C-Alkoxyalkyl bedeuten,
R3 Wasserstoff, 1-6C-Alkyl oder 3-7C-Alkoxyalkyl bedeutet,
R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxycarbonyl, 2-5C-Acyl, Amino oder Mono-oder Di-1-4C-alkylamino bedeuten,
R8 Aryl bedeutet und
R9 Aryl bedeutet, wobei
Aryl für einen Ring der Formel

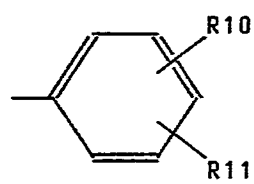

steht, worin R10 und R11 gleich oder verschieden sind und die Bedeutung Wasserstoff (H), 1-4C-Alkyl, 1-4C-Alkoxy, Halogen, Hydroxy oder Trifluormethyl haben,
und die Salze dieser Verbindungen.

5. Verbindungen der Formel Ib nach Anspruch 4, worin
Cy 3-Nitrophenyl, 2-Chlorphenyl, 2,3-Dichlorphenyl, 2-Trifluormethylphenyl oder Benzoxdiazolyl bedeutet,
A1 Ethylen ($-CH_2CH_2-$) bedeutet,
A2 Ethylen ($-CH_2CH_2-$) oder Ethylenoxyethylen ($-CH_2-CH_2-O-CH_2-CH_2-$) bedeutet,
R1 Methyl bedeutet,
R2 Methyl bedeutet,
R3 Methyl oder Ethyl bedeutet,
R8 Phenyl bedeutet und
R9 Phenyl bedeutet,
und ihre Salze.

6. Verbindungen ausgewählt aus der Gruppe bestehend aus
1,4-Dihydro-2,6-dimethyl-4-(3-nitrophenyl)-pyridin-3,5-dicarbonsäure-3-methyl-5-{2-[2-(4,4-diphenylpiperidyl-1)-ethoxi]-ethyl}-ester
(-)-1,4-Dihydro-2,6-dimethyl-4-(3-nitrophenyl)-pyridin-3,5-dicarbonsäure-3-methyl-5-{2-[2-(4,4-diphenylpiperidyl-1)-ethoxi]-ethyl}-ester
1,4-Dihydro-2,6-dimethyl-4-(3-nitrophenyl)-pyridin-3,5-dicarbonsäure-3-methyl-5-{2-[2-(2-(4,4-diphenylpiperidyl-1)-ethoxi-ethoxi]-ethyl}-ester
2-Amino-1,4-dihydro-6-methyl-4-(3-nitrophenyl)-pyridin-3,5-dicarbonsäure-3-ethyl-5-[3-(4,4-diphenylpiperidyl-1)-propyl]-ester
(-)-2-Amino-1,4-dihydro-6-methyl-4-(3-nitrophenyl)-pyridin-3,5-dicarbonsäure-3-ethyl-5-[3-(4,4-diphenylpiperidyl-1)-propyl]-ester
2-Amino-1,4-dihydro-6-methyl-4-(3-nitrophenyl)-pyridin-3,5-dicarbonsäure-3-ethyl-5-[5-(4,4-diphenylpiperidyl-1)-pentyl]-ester
2-Amino-4-(2,3-dichlorphenyl)-1,4-dihydro-6-methyl-pyridin-3,5-dicarbonsäure-3-ethyl-5-[3-(4,4-diphenylpiperidyl-1)-propyl]-ester
2-Amino-4-(4-benzo[c][1.2.5]oxdiazolyl)-1,4-dihydro-6-methyl-pyridin-3,5-dicarbonsäure-3-ethyl-5-[3-(4,4-diphenylpiperidyl-1)-propyl]-ester
2-Amino-4-(2-chlorphenyl)-1,4-dihydro-6-methyl-pyridin-3,5-dicarbonsäure-3-ethyl-5-[3-(4,4-diphenylpiperidyl-1)-propyl]-ester
2-Amino-1,4-dihydro-6-methyl-4-(2-trifluormethylphenyl)-pyridin-3,5-dicarbonsäure-3-ethyl-5-[3-(4,4-diphenylpiperidyl-1)-propyl]-ester
2-Amino-1,4-dihydro-6-methyl-4-(3-nitrophenyl)-pyridin-3,5-dicarbonsäure-3-methyl-5-[2-(4,4-diphenylpiperidyl-1)-ethyl]-ester
und ihre Salze.

7. Verfahren zur Herstellung der Verbindungen der Formel Ia nach Anspruch 2 und ihrer Salze, dadurch gekennzeichnet, daß man Amidine der Formel IIa

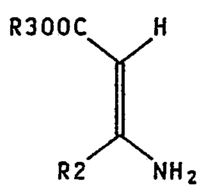

(IIa),

mit Benzylidencarbonsäurederivaten der Formel IIIa

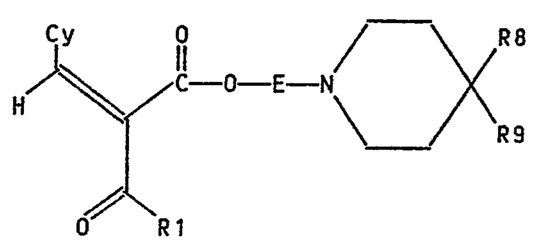

(IIIa),

als solche(n) oder in Form ihrer Salze umsetzt und gewünschtenfalls anschließend erhaltene Salze in die freien Basen oder erhaltene Basen in die Salze überführt, wobei Cy, E, R1, R2, R3, R4, R5, R8 und R9 die die in Anspruch 2 angegebenen Bedeutungen haben.

8. Verfahren zur Herstellung der Verbindungen der Formel Ib nach Anspruch 4 und ihrer Salze, dadurch gekennzeichnet, daß man

a) Zimtsäurederivate der Formel IIb

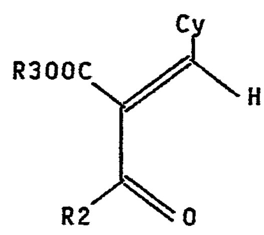

(IIb),

mit Enaminderivaten der Formel IIIb

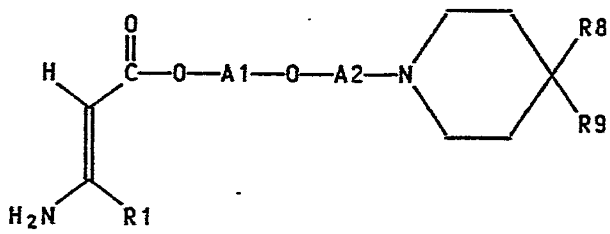

(IIIb),

oder

b) Zimtsäurederivate der Formel IIb mit Ammoniak und β-Ketocarbonsäurederivaten der Formel IV

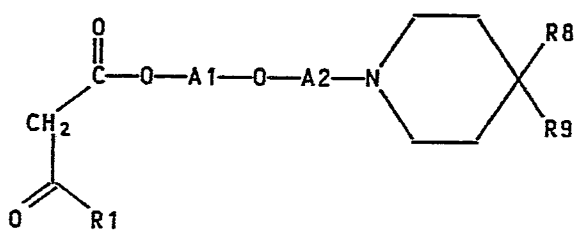

(IV),

oder

c) Enamine der Formel V

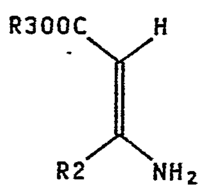

(V),

mit Benzylidencarbonsäurederivaten der Formel VI

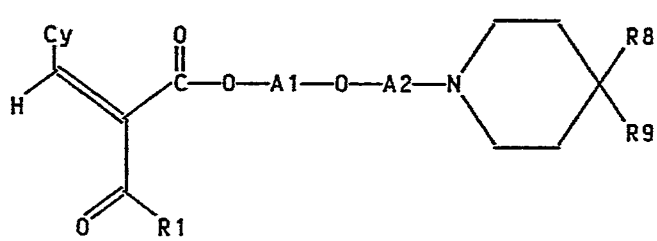

(VI),

oder

d) Ketoverbindungen der Formel VII

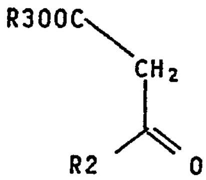

(VII),

mit Ammoniak und Benzylidencarbonsäurederivaten der Formel VI, oder

e) Aldehyde der Formel VIII

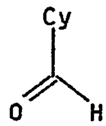

(VIII),

mit Enaminen der Formel V und β-Ketocarbonsäurederivaten der Formel IV, oder

f) Aldehyde der Formel VIII mit Enaminderivaten der Formel IIIb und Ketoverbindungen der Formel VII, oder

g) 1,4-Dihydropyridine der Formel IX

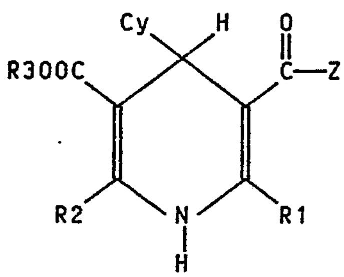

(IX),

mit Aminderivaten der Formel X

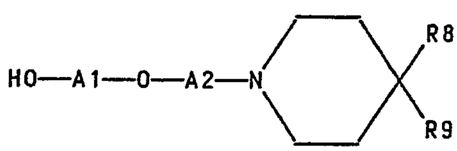

(X),

oder

h) 1,4-Dihydropyridinderivate der Formel XI

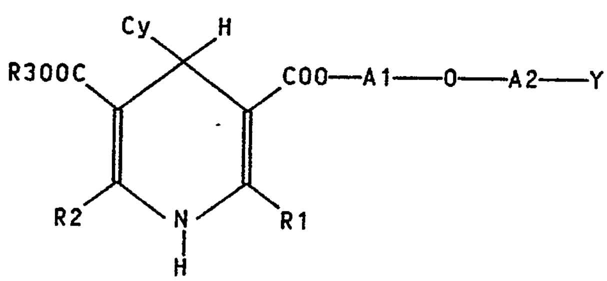

(XI),

mit Aminen der Formel XII

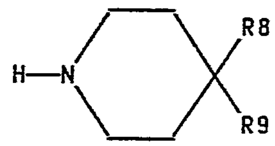

(XII),

als solche(n) oder in Form ihrer Salze umsetzt und gewünschtenfalls anschließend erhaltene Salze in die freien Basen oder erhaltene Basen in die Salze überführt, wobei Cy, A1, A2, R1, R2, R3, R4, R5, R8 und R9 die in Anspruch 4 angegebenen Bedeutungen haben, Z gemeinsam mit der Carbonylgruppe, woran es gebunden ist, eine Carboxylgruppe oder ein reaktives Carbonsäurederivat (z. B. ein Carbonsäurehalogenid) und Y eine Fluchtgruppe darstellt.

9. Arzneimittel enthaltend eine oder mehrere Verbindungen nach einem oder mehreren der Ansprüche 1 bis 6 und/oder ihre pharmakologisch verträglichen Salze.

10. Verbindungen nach einem oder mehreren der Ansprüche 1 bis 6 und ihre pharmakologisch verträglichen Salze zur Anwendung bei der Behandlung und/oder Prophylaxe von Hypertonie, koronaren Herzkrankheiten, peripheren und cerebralen Zirkulationsstörungen und/oder Krankheiten, die auf einer erhöhten Wasser-oder Natriumretention beruhen.

Europäisches Patentamt

# EUROPÄISCHER RECHERCHENBERICHT

Nummer der Anmeldung

EP 87 10 5748

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 176 956 (BYK GULDEN LOMBERG)<br>* Insgesamt * | 1,7-10 | C 07 D 211/90<br>C 07 D 405/14<br>C 07 D 409/14<br>C 07 D 413/14<br>C 07 D 417/14<br>A 61 K 31/14 //<br>C 07 D 211/14 |
| Y | EP-A-0 138 505 (TAKEDA)<br>* Beispiele 1-12; Ansprüche; Seiten 10-11 * | 1-3,7-10 | |
| A | EP-A-0 007 293 (HÄSCH)<br>* Beispiele 2,9,10,17,18,27; Seiten 19-20; Ansprüche * | 1,4,5,7-10 | |
| A | GB-A-2 158 065 (BRISTOL MYERS)<br>* Seiten 10-12; Beispiele 58-59; Ansprüche * | 1,4,5,7-10 | **RECHERCHIERTE SACHGEBIETE (Int. Cl.4)** |
| | ----- | | C 07 D 211/00<br>C 07 D 405/00<br>C 07 D 409/00<br>C 07 D 413/00<br>C 07 D 417/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 14-07-1987 | NUYTS A.M.K.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82